(19) Europäisches Patentamt
European Patent Office
Office européen des brevets

(11) Publication number: 0 086 554
A1

(12) # EUROPEAN PATENT APPLICATION

(21) Application number: 83300174.6

(22) Date of filing: 13.01.83

(51) Int. Cl.³: C 07 D 307/62, C 07 D 407/04, C 07 D 405/12, C 07 D 405/14, A 61 K 31/375

(30) Priority: 15.01.82 US 339344

(43) Date of publication of application: 24.08.83 Bulletin 83/34

(84) Designated Contracting States: AT BE CH DE FR GB IT LI LU NL SE

(71) Applicant: ELI LILLY AND COMPANY, 307, East McCarty Street, Indianapolis Indiana 46285 (US)

(72) Inventor: Koppel, Gary Allen, 7823 Sunset Lane, Indianapolis Indiana 46240 (US)
Inventor: Barton, Russell Lavern, 3475 Beluga Lane Apt. 1-B, Indianapolis Indiana 46226 (US)
Inventor: Bewley, Jesse Robert, 4306, Hoyt Avenue, Indianapolis Indiana 46203 (US)
Inventor: Briggs, Stephen Lyle, Rural Route No. 1 Box 483, Clayton Indiana 46118 (US)
Inventor: Parton, Joseph Wayne, Rural Route No. 4, Box 360, Greenfield Indiana 46140 (US)

(74) Representative: Crowther, Terence Roger et al, Erl Wood Manor, Windlesham Surrey GU20 6PH (GB)

(54) **Ascorbic acid ethers and related compounds.**

(57) Ethers of ascorbic acid, isoascorbic acid, dihydroascorbic acid, dihydroisoascorbic acid, deoxyascorbic acid, and scorbamic acid, and ketals and acetals thereof, having angiogenesis and arthritis inhibiting properties, are provided.

EP 0 086 554 A1

# Ascorbic Acid Ethers and Related Compounds

The present invention relates to compounds that display angiogenesis and arthritis inhibiting activity.

Angiogenesis refers to the process of new blood vessel development. Proliferation of new blood vessels is involved in various disease states, such as tumor growth, retinopathy, psoriasis and rheumatoid arthritis (pannus formation).

Naturally occurring angiogenesis inhibitors have been isolated from cartilage by several research groups, and it has been shown that such angiogenesis inhibitors inhibit various enzymes, such as collagenase. T.H. Maugh II, Angiogenesis Inhibitors Link Many Diseases, Science, 212: 1374-75 (1981). It has also been reported that cartilage angiogenesis inhibitors inhibit proliferation of osteoclasts, the cells that are responsible for resorption of bone.

Angiogenesis inhibitors that have been isolated from cartilage and other natural sources are proteins. These have only been available in very small quantities, and have not been fully characterized.

It would be desirable to provide angiogenesis and arthritis inhibiting compounds of known structure that can be made in commercial quantities.

This invention provides compounds that exhibit angiogenisis and arthritis inhibiting activity. More particularly, the invention provides a compound of the formula (I)

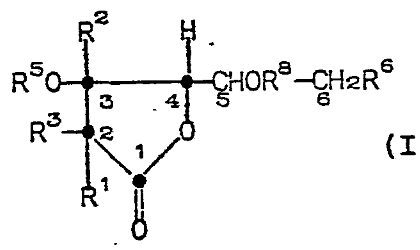

(I)

wherein:

$R^1$ and $R^2$ are both hydrogen or combine to form a second bond between $C_2$ and $C_3$,

$R^3$ is OH, $NH_2$ or $OR^4$,

$R^4$ and $R^5$ are independently selected from

$(C_8-C_{22})$alkyl,
$-CH_2(C_2-C_{12})$alkenyl,
$-CH_2(C_2-C_{12})$alkynyl,
$-(C_1-C_{21})$alkyl-X-$(C_1-C_{21})$alkyl,

wherein X is O, CO, S, NH, $N(C_1-C_5)$alkyl, SO, or $SO_2$, or

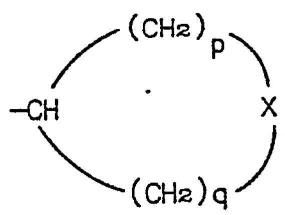

wherein X is as defined above and the sum of p and q is 1-6, said $R^4$ and $R^5$ groups being unsubstituted or optionally substituted with one or two groups independently selected from Cl, Br, F, I, $(C_1-C_5)$-alkoxycarbonyl, phenoxy, OH, $CF_3$, $(C_1-C_5)$alkoxy, nitro, -CN, $-SO_3H$, $-PO_3H_2$, di-$(C_1-C_5)$-alkylamino, or phthalimido,

$R^6$ is H, F or $OR^7$;

$R^7$ and $R^8$, taken singly, are independently selected from H, $(C_1-C_{12})$alkyl, and benzyl, or, taken together, are

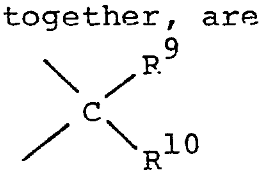

wherein $R^9$ and $R^{10}$ are independently selected from

H,

a $(C_1-C_{10})$alkyl group that is optionally substituted with halo, phenyl, or substituted phenyl, said optional phenyl substituents being one or two groups independently selected from halo, hydroxy, $(C_1-C_5)$alkoxy, nitro, $CF_3$, and $(C_1-C_5)$alkyl, or

optionally substituted phenyl, wherein said optional phenyl substituents are as defined above,

provided that only one of $R^9$ and $R^{10}$ can be H, or a pharmaceutically acceptable salt thereof.

The invention also provides a process for making the compounds of formula (I) which comprises the steps of

(a) reacting a compound of the formula (II)

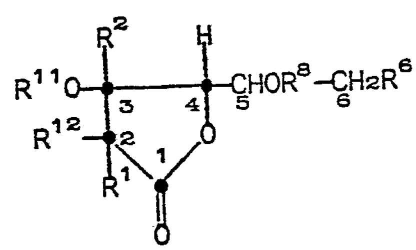

(II)

wherein $R^2$, $R^6$ and $R^8$ are as defined above, $R^{11}$ is H or $R^5$ as defined above, and

$R^{12}$ is OH, $OR^4$ as defined above, or $NH_2$, provided that $R^{12}$ is OH if $R^{11}$ is other than H, with an alkylating reagent of the formula $R^4Z$ or $R^5Z$, where Z is halogen or a halogen-like leaving group such as p-tosyl, mesyl, or a dialkyl sulfate, and $R^4$ and $R^5$ are as defined above, in the presence of a base such as an alkali metal lower alkanolate, in an inert solvent, or

(b) when $R^{11}$ is other than H, $R^6$ is $OR^7$, and $R^7$ and $R^8$ combine to form

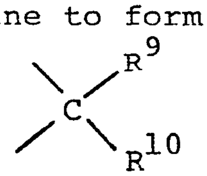
,

subjecting the compound of formula II to acid hydrolysis to produce a product of formula I wherein $R^7$ and $R^8$ are hydrogen.

Another aspect of the invention is that it provides a compound of the formula (I) wherein:

$R^1$ and $R^2$ are both hydrogen or combine to form a second bond between $C_2$ and $C_3$,

$R^3$ is OH, $NH_2$ or $OR^4$,

$R^4$ and $R^5$ are independently selected from

$(C_1-C_{22})$alkyl,

$-CH_2(C_2-C_{12})$alkenyl,

$-(CHR^{13})_m-Y-R^{14}$ wherein m is 0 to 12, Y is O, S, or a direct bond, $R^{13}$ is H or $(C_1-C_5)$alkyl, and $R^{14}$ is $(C_3-C_8)$cycloalkyl, $(C_3-C_8)$cycloalkenyl, $(C_7-C_{12})$bicycloalkyl, $(C_7-C_{12})$-bicycloalkenyl, or aryl,

$-CH_2(C_2-C_{12})$alkynyl,

$-(C_1-C_{21})$alkyl-X-$(C_1-C_{21})$alkyl, wherein X is O, CO, S, NH, $N(C_1-C_5)$alkyl, SO, or $SO_2$, or

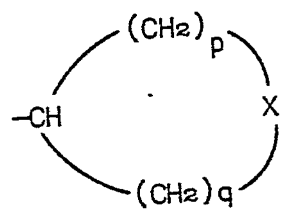

wherein X is as defined above and the sum of p and q is 1-6, said $R^4$ and $R^5$ groups being unsubstituted or optionally substituted with one or two groups independently selected from Cl, Br, F, I, $(C_1-C_5)$-alkoxycarbonyl, phenoxy, OH, $CF_3$, $(C_1-C_5)$alkoxy, nitro, -CN, $-SO_3H$, $-PO_3H_2$, di-$(C_1-C_5)$-alkylamino, or phthalimido,

$R^6$ is H, F or $OR^7$;

$R^7$ and $R^8$, taken singly, are independently selected from H, $(C_1-C_{12})$alkyl, and benzyl, or, taken together, are

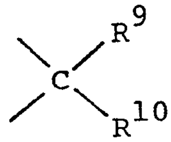

wherein $R^9$ and $R^{10}$ are independently selected from

H,

a $(C_1-C_{10})$alkyl group that is optionally substituted with halo, phenyl, or substituted phenyl, said optional phenyl substituents being one or two groups independently selected from halo, hydroxy, $(C_1-C_5)$alkoxy, nitro, $CF_3$, and $(C_1-C_5)$, or

optionally substituted phenyl, wherein said optional phenyl substituents are as defined above,

provided that only one of $R^9$ and $R^{10}$ can be H, and the pharmaceutically acceptable salts thereof, for use as a pharmaceutical.

The invention is also embodied in a pharmaceutical formulation including, as an active ingredient, a compound of the formula (I) wherein:

$R^1$ and $R^2$ are both hydrogen or combine to form a second bond between $C_2$ and $C_3$,

$R^3$ is OH, $NH_2$ or $OR^4$,

$R^4$ and $R^5$ are independently selected from

$(C_1-C_{22})$alkyl,

$-CH_2(C_2-C_{12})$alkenyl,

$-(CHR^{13})_m-Y-R^{14}$ wherein m is 0 to 12, Y is O, S, or a direct bond, $R^{13}$ is H or

$(C_1-C_5)$alkyl, and $R^{14}$ is $(C_3-C_8)$cycloalkyl, $(C_3-C_8)$cycloalkenyl, $(C_7-C_{12})$bicycloalkyl, $(C_7-C_{12})$-bicycloalkenyl, or aryl,

$-CH_2(C_2-C_{12})$alkynyl,

$-(C_1-C_{21})$alkyl-X-$(C_1-C_{21})$alkyl,

wherein X is O, CO, S, NH, $N(C_1-C_5)$alkyl, SO, or $SO_2$, or

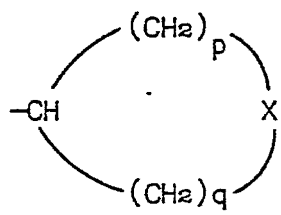

wherein X is as defined above and the sum of p and q is 1-6, said $R^4$ and $R^5$ groups being unsubstituted or optionally substituted with one or two groups independently selected from Cl, Br, F, I, $(C_1-C_5)$-alkoxycarbonyl, phenoxy, OH, $CF_3$, $(C_1-C_5)$alkoxy, nitro, -CN, $-SO_3H$, $-PO_3H_2$, di-$(C_1-C_5)$-alkylamino, or phthalimido,

$R^6$ is H, F or $OR^7$;

$R^7$ and $R^8$, taken singly, are independently selected from H, $(C_1-C_{12})$alkyl, and benzyl, or, taken together, are

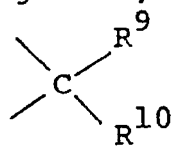

wherein $R^9$ and $R^{10}$ are independently selected from

H,

a $(C_1-C_{10})$alkyl group that is optionally substituted with halo, phenyl, or substituted phenyl, said optional phenyl substituents being one or two groups independently selected from halo, hydroxy, $(C_1-C_5)$alkoxy, nitro, $CF_3$, and $(C_1-C_5)$, or

optionally substituted phenyl, wherein said optional phenyl substituents are as defined above,

provided that only one of $R^9$ and $R^{10}$ can be H, and the pharmaceutically acceptable salts thereof, in combination with one or more pharmaceutically acceptable excipients.

Formula (I) represents ethers of ascorbic or isoascorbic acid when $R^1$ and $R^2$ combine to form a second bond and $R^6$ is OH. It represents ethers of dihydroascorbic or dihydroisoascorbic when $R^1$ and $R^2$ are both hydrogen and $R^6$ is OH. It represents ethers of scorbamic acid when $R^1$ and $R^2$ combine to form a second bond, $R^3$ is $NH_2$ and $R^6$ is OH. It represents ethers of deoxyascorbic acid when $R^1$ and $R^2$ combine to form a second bond and $R^6$ is H or F.

Ascorbic acid and isoascorbic acid can be represented by the formula (III)

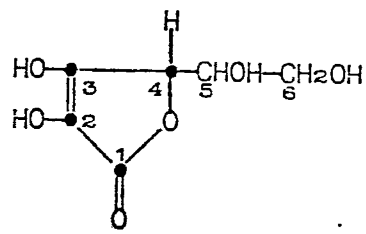

(III)

In formula (III), $C_4$ and $C_5$ are asymmetric carbons; thus, formula (III) represents 4 stereoisomers of 3-ketohexuronic acid lactone (enol form). The absolute stereochemical configuration of these 4 stereoisomers and the corresponding trivial names are as follows.

$C_4(R)C_5(S)$ 3-ketohexuronic acid lactone (enol form), known as L-ascorbic acid

$C_4(R)C_5(R)$ 3-ketohexuronic acid lactone (enol form), known as D-isoascorbic acid

$C_4(S)C_5(R)$ 3-ketohexuronic acid lactone (enol form), known as D-ascorbic acid

$C_4(S)C_5(S)$ 3-ketohexuronic acid lactone (enol form), known as L-isoascorbic acid

L-ascorbic acid (vitamin C) can also be named as 3-oxo-L-gulofuranolactone (enol form) and it is a derivative of L-gulofuranose. Likewise, D-ascorbic acid is a derivative of D-gulofuranose. The isoascorbic acids are derivatives of glucofuranose. Compounds according to the above formula can be named systematically as derivatives of 2-oxo-3,4-dihydroxy-5-(1,2-dihydroxyethyl)-2,5-dihydrofuran; ie. L-ascorbic acid would be $C_4$(R)$C_5$(S) 2-oxo-3,4-dihydroxy-5-(1,2-dihydroxyethyl)-2,5-dihydrofuran. However, the hexuronic acid terminology will be used throughout in referring to compounds according to formula (III).

Scorbamic and isoscorbamic acids are represented by the formula (IV)

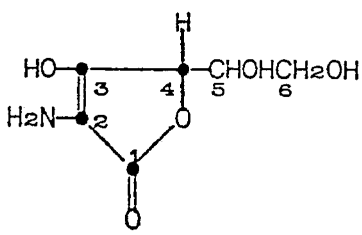

(IV)

Compounds according to formula (IV) are named systematically as 2-oxo-3-amino-4-hydroxy-5-(1,2-dihydroxyethyl)-2,5-dihydrofuran. However, to be consistent with the generic name for compounds of formula III, these compounds will be named as isomers of

3-keto-2-aminohexuronic acid lactone (enol form). Two asymmetric carbons $C_4$ and $C_5$ are likewise present in the above molecule and the 4 stereoisomers represented thereby have the following absolute configurations.

$C_4$(R)$C_5$(S) 3-keto-2-aminohexuronic acid lactone (enol form), L-scorbamic acid; $C_4$(R)$C_5$(R) 3-keto-2-aminohexuronic acid lactone (enol form), D-isoscorbamic acid; $C_4$(S)$C_5$(R) 3-keto-2-aminohexuronic acid lactone (enol form), D-scorbamic acid; $C_4$(S)$C_5$(S) 3-keto-2-aminohexuronic acid lactone (enol form), L-isoscorbamic acid. The terms "ascorbic acid" and "scorbamic acid" as used herein includes all 4 stereoisomers unless a given absolute configuration is specified.

6-Deoxy ascorbic acids can be represented by the following formula (V)

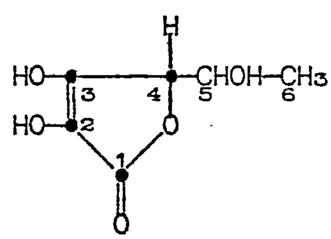

(V)

As in ascorbic acid, $C_4$ and $C_5$ are asymmetric centers giving rise to 4 stereoisomers. The compounds of formula (IV) will be referred to as 6-deoxyascorbic acids. An alternate name is 3-keto-6-deoxyhexuronic acid lactone (enol form).

Dihydroascorbic acid and dihydroisoascorbic acid can be represented by the following formula (VI)

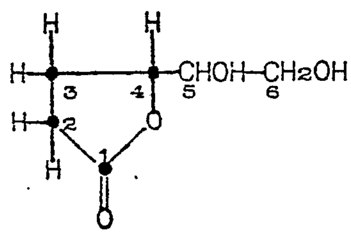

(VI)

Systematically, such compounds are named as 2,3-dihydroxy-5-(1-hydroxyethyl)tetrahydrofurans. As can be seen, there are 4 asymmetric centers in dihydroascorbic acid -- at $C_2$, $C_3$, $C_4$ and $C_5$. There are thus $2^4$=16 stereoisomers represented by the above formula plus ethers, acetals and ketals thereof active as angiogenesis inhibitors falling within the ambit of this invention.

It will be recognized that when $R^6$ is $-OR^7$ and $R^7$ and $R^8$ are taken together to form

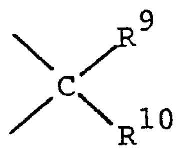

the resulting structure is a ketal when $R^9$ and $R^{10}$ are both other than H and an acetal when $R^9$ or $R^{10}$ is H. For example, if $R^9$ is methyl and $R^{10}$ is ethyl, the resulting structure is a ketal of methyl ethylketone formed with the vicinal hydroxyls at C-5 and C-6.

In the above formulas, the following acyclic and cyclic aliphatic radicals exemplify the terms $R^4$, $R^5$, $R^7$, $R^8$, $R^9$, $R^{10}$, $R^{13}$, and $R^{14}$:
Ethyl, n-propyl, isopropyl, sec.-butyl, n-butyl, isobutyl, iso-amyl, t-amyl, n-amyl, 2-pentyl, 3-pentyl, 3-methyl-2-butyl, 2-hexyl, 1-hexyl, 3-hexyl, 4-methyl-1-pentyl, 3-methyl-1-pentyl, 3-methyl-2-pentyl, neopentyl, 3,3-dimethyl-1-butyl, 3,3-dimethyl-1-pentyl, 3,3,4-trimethyl-1-pentyl, 2,2,4-trimethyl-1-pentyl, 2,4,4-dimethyl-2-pentyl, iso-octyl, isoheptyl, n-heptyl, 2-heptyl, 3-heptyl, 4-heptyl, 2-octyl, 3-octyl, 4-octyl, 2-methyl-2-butenyl, 2-methyl-3-propenyl, allyl, methallyl, crotyl, cyclobutyl, cyclopropyl, cyclopentyl, cyclohexyl, 3-methylcyclopentyl, 3-ethylcyclohexyl, cycloheptyl, 4-methylcycloheptyl, 2-methylcycloheptyl, cyclooctyl, 1-cyclohexenyl, 2-cyclohexenyl, 3-cyclohexenyl, 2-cyclopentenyl, 3-ethyl-2-cyclohexenyl, 2-cyclopentenyl, 4-methyl-2-cycloheptenyl, 4-cyclooctenyl, 2-bromoethyl, 2-iodoethyl, benzyl,

o-chlorobenzyl, m-bromobenzyl, 2,4-dichlorobenzyl, p-nitrobenzyl, 2-iodobenzyl, 4-fluorobenzyl, 1-methyl-4-trifluoromethylbenzyl, 2-methyl-2-chlorobenzyl, m-ethoxybenzyl, bicyclo[2.2.1]heptanyl, bicyclo[2.2.1]heptanylmethyl, bicyclo[2.2.1]heptenyl, bicyclo[2.2.1]heptenylmethyl, bicyclo[3.3.0]octanyl, bicyclo[2.4.0]octenyl, bicyclo[3.3.0]octenylethyl, bicyclo[3.3.1]nonyl, bicyclo[3.3.1]nonylmethyl, bicyclo[3.3.1]nonenyl, bicyclo[3.3.1]nonenylmethyl, bicyclo[3.1.1]heptanyl, bicyclo[3.3.1]heptanylpropyl, bicyclo[3.1.1]heptenyl, bicyclo[3.1.1]heptenylmethyl, bicyclo[4.2.0]octanyl, bicyclo[4.2.0]octenyl, bicyclo[4.2.0]octenylmethyl, 3-methylbicyclo[4.2.0]octenyl, bicyclo[4.3.1]-cyclododecyl, bicyclo[4.3.1]cyclodecylmethyl, bicyclo[5.3.0]cyclodecenyl, 5-methylbicyclo[5.3.0]-cyclododecenyl, bicyclo[3.2.0]heptanyl, bicyclo[3.2.0]heptanylethyl, bicyclo[3.2.0]heptenyl, bicyclo[3.2.0]heptenylmethyl, bicyclo[4.1.0]heptanyl, bicyclo[4.1.0]heptanylmethyl, bicyclo[4.1.0]heptenyl, bicyclo[4.1.0]heptenylmethyl, 2-isopropylbicyclo[4.1.0]heptenyl, 2-isopropyl-3-methyl-bicyclo[3.1.0]hexenyl, n-hendecyl, n-dodecyl, n-tridecyl, n-tetradecyl, n-pentadecyl, n-hexadecyl, n-heptadecyl, n-octadecyl, n-eicosyl, n-docosyl, 2-tetradecyl, 4-tetradecyl, 6-tetradecyl, 7-tetradecyl, 7-hexadecyl, 8-hexadecyl, 9-octadecyl, 2-octyldodecyl, 3,7,11-trimethyldodecyl, tetrahydrogeranyl, 12-methyltridecyl, myristoleyl,

myristeladyl, oleyl, linoleyl, 12-methyltridecen-9-yl etc.

The compounds of formula (I) where $R^1$ and $R^2$ combine to form a double bond and $R^3$ is $-OR^4$ can be prepared by reacting ascorbic acid, or isoascorbic acid or a ketal or acetal of the formula (VII)

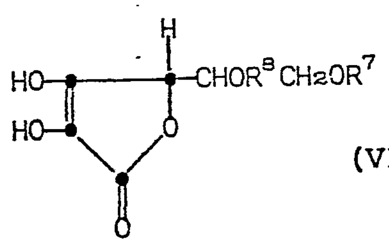

(VII)

wherein $R^7$ and $R^8$ are as previously defined, with one or two moles of a base such as an alkali metal lower alkanolate plus an alkylating agent wherein $R^4$ or $R^5$ is as defined above and X is a leaving group (a group labile to nucleophillic displacement) such as halogen, (Cl, I, Br) or is a halogen-like leaving group; i.e., p-tosyl (p-toluene sulfonate) or mesyl (methane sulfonate). A dialkyl sulfate ($R_2SO_4$) can also be used. If one mole each of base and alkylating agent is used, the above reaction produces an ether group at C-3. If it is desired to prepare a

diether, with identical ether groups at both C-2 and C-3, two moles of the base and two moles of alkylating agent are used. The resulting compound is a diether of formula (VIII)

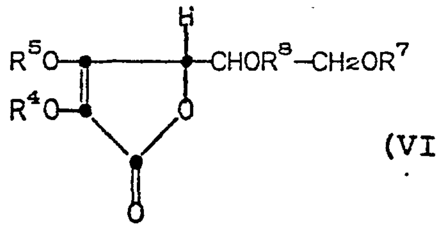

(VIII)

in which $R^4$ and $R^5$ represent the same group. If a diether is desired in which $R^4$ and $R^5$ are different groups, a monoether of formula (II)

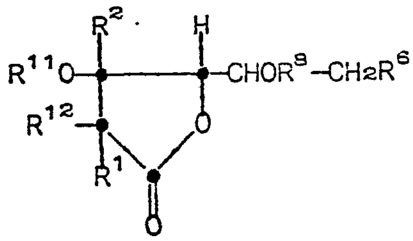

(II)

in which $R^{11}$ is ethyl, for example, and $R^{12}$ is -OH is reacted with an alkylating reagent $R^4X$ wherein

$R^4$ is other than ethyl, and one mole of base. Depending on the relative reactivities of the C-2 and C-3 hydroxyl groups and of the alkylating agent, a certain amount of reaction takes place at C-2 even if only one mole of alkylating agent is used, and it is desired to produce a C-3 ether only. The mixture of mono and diethers thus formed can be readily separated, as by chromatography. There is also a possibility, where $R^7$ and $R^8$ are hydroxyls, of partially alkylating one of these and forming a diether at C-3 and C-5, for example such diethers can also be separated by chromatography.

The above reactions are carried out in an inert mutual solvent, such as DMSO (dimethylsulfoxide), DMF (N,N-dimethylformamide), acetonitrile, nitromethane, diethylsulfoxide. The reactions can be carried out at any convenient temperature in the range 0° to 80°C., but are ordinarily carried out at ambient temperature. The preferred base is sodium methoxide.

Under certain circumstances, particularly where there is a competing reaction with the C-5 or C-6 hydroxy, the L-ascorbic acid ethers can be prepared in exceptionally pure form by alkylating L-ascorbic acid 5,6-acetonide (I where $R^7$ and $R^8$ in formula VII together form a 1-methylethylidine group) and then removing the ketal group by treatment with acid --acetic, 1% HCl etc. This procedure

selectively hydrolyzes the ketal group without affecting the ether groups at C-2 and/or C-3.

The ketal and acetal starting materials of formula (VII) are prepared by standard procedures such as a reaction in dioxane or other inert, anhydrous, mutual solvent in the presence of an excess of a Lewis acid; e.g., zinc chloride or the like.

Scorbamic ethers, ketals, and acetals are prepared in similar fashion to ascorbic and isoascorbic acid ethers, etc., although it should be recognized that ethers can form at only C-3 on the ring since C-2 carries an amine function.

Compounds of formula (I) wherein $R^1$ and $R^2$ are both hydrogen are prepared directly from dihydroascorbic acid, using the procedures exemplified above for ascorbic acid and isoascorbic acid.

This invention is further illustrated by the following specific examples.

Example 1

3-O-n-Butyl-L-ascorbic acid (Compound 1)

A reaction mixture was prepared containing the following ingredients: 33 g. of L-ascorbic acid, 10.2 g. of sodium methoxide, 34.5 g. of n-butyl iodide and 250 ml. of DMSO. The reaction mixture was stirred at ambient temperature and the progress of the reaction monitored by thin-layer chromatography. After 24 hours, the reaction mixture was added to 500 ml. of ethyl acetate. 3-O-n-Butyl-L-ascorbic acid formed in the above reaction precipitated and was

separated by filtration. Further precipitation was caused by adding 300 ml. of toluene to the filtrate. These crystals were also collected and the combined crystals dissolved in 500 ml. of methanol. (weight = about 20 g.) The collected yellow crystals were dissolved in 500 ml. of methanol and 45 g. of silica gel was added to this solution. The solution was evaporated to dryness in vacuo.

A chromatographic column was prepared in the following manner: 100 g. of silica 60 were mixed with 500 ml. of hexane. This mixture was placed in a glass chromatographic column containing a glass wool plug covered by a 3-5 mm. layer of sea sand under a nitrogen atmosphere. The silica gel was allowed to compact for a period of about 20 minutes. Another 2-4 mm. layer of sea sand was added. It was necessary in both instances that the sea sand layers be leveled. Next the silica-precipitate dried mixture was mixed with hexane and this mixture added carefully to the top of the column. Next, about a 5 g. quantity of silica in hexane was added. The column was again placed under nitrogen atmosphere for 15-20 minutes while the two new silica layers were compacting. Finally, a layer of sand (3-6 mm. thick) was added.

The chromatogram was developed as follows: 8 liters of a 1:1 ethyl acetate/toluene mixture were passed through the column. No substantial amounts of the desired L-ascorbic acid ether were obtained. Next, 4 liters of a 3:1 ethyl acetate/toluene eluate

were passed through the column, thereby eluting most of the desired ether. Evaporation of the solvent yielded 3-O-n-butyl-L-ascorbic acid having the following analysis:

Calculated: C, 51.72; H, 6.94;
Found: C, 51.45; H, 6.72.

Mass spectrum: peaks at 232 (molecular ion), 172, 145, 100, 85, 71, 57, 41, 29.

Other compounds prepared by the above procedure include:

3-O-(2,6-dichlorobenzyl)-L-ascorbic acid (Compound 2)

Analysis calculated: C, 46.59; H, 3.61; Cl, 21.16;
Found: C, 46.34; H, 3.53; Cl, 20.88.

Mass spectrum: peaks at 428 (molecular ion), 192.

3-O-allyl-L-ascorbic acid (Compound 3)

Mass spectrum: peaks at 216 (molecular ion) 156, 58, 40.

2,3-di-(O-allyl)-L-ascorbic acid (Compound 4)

Analysis calculated: C, 56.25; H, 6.29;
Found: C, 56.12; H, 5.93.

Mass spectrum: peaks at 256 (molecular ion) 216, 174, 58, 40.

3-O-n-dodecyl-L-ascorbic acid (Compound 5)

Yield = 7.183 g. from 33.0 g. of L-ascorbic acid

Mass spectrum: peaks at 344 (molecular ion) 284, 177, 145, 116, 100, 85, 71, 61, 57, 43, and 29.

3-O-(3-bromobenzyl)-L-ascorbic acid (Compound 6)

Yield = 3.986 g. from 17.6 g. of L-ascorbic acid

Analysis calculated: C, 45.24; H, 3.80; Br, 23.15;
Found: C, 45.45; H, 3.57; Br, 22.94.

pKa = 10.50

3-O-(3-fluorobenzyl)-L-ascorbic acid (Compound 7)

Yield = 4.194 g. from 23.3 g. of L-ascorbic acid

Analysis calculated: C, 54.93; H, 4.61; F, 6.68;
Found: C, 55.07; H, 4.42; F, 6.49.

Mass spectrum: molecular ion at 284

3-O-(10-carboxy-n-decyl)-L-ascorbic acid (Compound 8)

Analysis calculated: C, 56.66; H, 7.83;
Found: C, 56.93; H, 7.55.

Mass spectrum: peaks at 361 (molecular ion), 58.

3-O-n-pentadecyl-L-ascorbic acid (Compound 9)

Yield = 3.6 g. from 15.2 g. of L-ascorbic acid

2,3-di-(O-n-pentadecyl)-L-ascorbic acid (Compound 10) (isolated from the same reaction mixture as the monoether)

Analysis calculated: C, 72.49; H, 11.48;
Found: C, 72.64; H, 11.28.

Yield = 1.26 g.

3-O-(2-bromoethoxyethyl)-L-ascorbic acid (Compound 11)

Analysis calculated: C, 36.72; H, 4.62; Br, 24.43;
Found: C, 36.46; H, 4.92; Br, 24.23.

Mass spectrum: peaks at 328, 326, 382, 58

3-O-(3-phenoxypropyl)-L-ascorbic acid (Compound 12)

Analysis calculated: C, 58.06; H, 5.85;
Found: C, 58.17; H, 5.59.

Mass spectrum: peaks at 310 (molecular ion).

3-O-(2-phthalimidoethyl)-L-ascorbic acid (Compound 13)

Mass spectrum: peaks at 349 (molecular ion) 193, 174, 161, 148, 130, 102, 76, 44, 28.

3-O-n-hexadecyl-L-ascorbic acid (Compound 14)

Analysis calculated: C, 65.97; H, 10.07; O, 23.97;
Found: C, 66.24; H, 9.84; O, 24.07.

Titration: pKa = 11.10.

Infrared spectrum: ν at 1750, 1695, 1680 $cm^{-1}$

2,3-di-(O-n-hexadecyl)-L-ascorbic acid (Compound 15)

Analysis calculated: C, 73.03; H, 11.61; O, 15.36;
Found: C, 72.92; H, 11.88; O, 15.07.

Infrared spectrum: ν at 1740, 1680 $cm^{-1}$

Titration-no titratable group

3-O-n-heptadecyl-L-ascorbic acid (Compound 16)

Analysis calculated: C, 66.63; H, 10.21;
Found: C, 66.37; H, 9.93.

Infrared spectrum: ν at 1760, 1710, 1695 $cm^{-1}$H

Mass spectrum: peaks at 414 (molecular ion), 354, 177, 116, 97.

3-O-n-octadecyl-L-ascorbic acid (Compound 17)

Analysis calculated: C, 67.26; H, 10.35;
Found: C, 67.42; H, 10.37.

Infrared spectrum: ν at 1757, 1705, 1690 $cm^{-1}$

Mass spectrum: peaks at 428 (molecular ion), 297, 98, 63

2,3-di-n-octadecyl-L-ascorbic acid (Compound 18)

Analysis calculated: C, 74.07; H, 11.84;
Found: C, 74.34; H, 12.07.

Infrared spectrum: ν at 1770, 1680 $cm^{-1}$

<u>3-O-n-icosyl-L-ascorbic acid</u> (Compound 19)

Mass spectrum: 456 (molecular ion)

Infrared spectrum: ν at 1690, 1705, 1758, 3436 $cm^{-1}$

<u>3-O-benzyl-L-ascorbic acid</u> (Compound 20)

Analysis calculated: C, 58.65; H, 5.30;

Found: C, 58.53; H, 5.60;

Mass spectrum: peaks at 266 (molecular ion), 228, 166, 148, 107, 91

Infrared spectrum: ν at 1760, 1695 $cm^{-1}$

<u>3-O-(3-chlorobenzyl)-L-ascorbic acid</u> (Compound 21)

Analysis calculated: C, 51.93; H, 4.36; Cl, 11.79

Found: C, 51.77; H, 4.10; Cl, 12.09.

Infrared spectrum: ν at 1740, 1690, 1680 $cm^{-1}$.

Mass spectrum: peaks at 300 (molecular ion) 240, 147, 125, 89

<u>3-O-(4-chlorobenzyl)-L-ascorbic acid</u> (Compound 22)

Analysis calculated: C, 51.93; H, 4.36; Cl, 11.79;

Found: C, 51.71; H, 4.21; Cl, 11.86.

Infrared spectrum: ν at 1755, 1695 $cm^{-1}$

$^{13}C$ nmr: δ at 170.36, 150.09, 135.62, 132.82, 129.53, 129.42, 119.73, 74.63, 71.06, 68.58, 61.82.

3-O-(3-trifluoromethylbenzyl)-L-ascorbic acid (Compound 23)

Analysis calculated: C, 50.31; H, 3.92; F, 17.05;

Found: C, 50.59; H, 3.40; F, 17.00.

Infrared spectrum: ν at 1755, 1695 $cm^{-1}$

Mass spectrum: peaks at 334 (molecular ion), 295, 274, 228, 159.

$^{13}C$ nmr: δ at 170.32, 149.94, 119.85, 74.66, 71.14, 68.62, 61.81.

3-O-(3-methylbenzyl)-L-ascorbic acid (Compound 24)

Analysis calculated: C, 60.00; H, 5.75;

Found: C, 60.21; H, 5.82.

Infrared spectrum: ν at 1740, 1685, 1675 $cm^{-1}$

Mass spectrum: peaks at 280 (molecular ion) 262, 186, 162, 134, 105, 91.

3-O-(2,5-dimethylbenzyl)-L-ascorbic acid (Compound 25)

Analysis calculated: C, 61.22; H, 6.17;

Found: C, 61.02; H, 6.22.

Infrared spectrum: ν at 1755, 1695 $cm^{-1}$

Mass spectrum: peaks at 294 (molecular ion), 176, 158, 147, 131, 119, 91.

3-O-n-octadecyl-D-ascorbic acid (Compound 26)

Analysis calculated: C, 67.3; H, 10.4;

Found: C, 67.1; H, 10.4.

Infrared spectrum: ν at 1700, 1755, 2840, 2905 $cm^{-1}$

Mass spectrum: molecular ion at 428

Titration: pKa = 11.00.

3-O-n-octadecyl isoascorbic acid (Compound 27)

Analysis calculated: C, 67.3; H, 10.4;

Found: C, 66.8; H, 9.3.

Titration: pKa = 11.60.

Mass spectrum: molecular ion at 428

Infrared spectrum: ν at 1695, 1755, 2840, 2905 $cm^{-1}$

3-O-(2-methylbenzyl)-L-ascorbic acid (Compound 28)

Analysis calculated: C, 60.00; H, 5.8; O, 34.2;

Found: C, 59.9; H, 5.5; O, 34.1.

Titration: pKa = 10.78.

Mass spectrum: $M^+$ = 280

Infrared spectrum: ν at 1685, 1750, 3370 $cm^{-1}$

2-O-(3-dimethylaminopropyl)-3-O-n-octadecyl-L-ascorbic acid hydrochloride (Compound 29)

Analysis calculated: C, 63.31; H, 10.26; N, 2.55; Cl, 6.44;

Found: C, 63.10; H, 10.13; N, 2.69; Cl, 6.66.

Infrared spectrum: ν at 1762, 1675 $cm^{-1}$

Titration: pKa = 8.0

Mass spectrum: peaks at 513, 482, 415, 344, 260, 201, 160.

3-O-(2-chlorobenzyl)-L-ascorbic acid (Compound 30)

Infrared spectrum: ν at 1690, 1760 $cm^{-1}$

Mass spectrum: major peak at 300

## Example 2

3-O-n-butyl-5,6-O-(benzylidene)-L-ascorbic acid (Compound 31)

Following the procedure of Example 1, a reaction mixture was prepared from 150 ml. of DMSO, 15 g. of 5,6-O-benzylidene-L-ascorbic acid (Compound 33), 3.24 mg. of sodium methoxide and 10.5 g. of n-butyl iodide. The reaction mixture was stirred at ambient temperature for about 72 hours at which time TLC indicated that the reaction had gone substantially to completion. The reaction mixture was extracted with 600 ml. of ethyl acetate and the ethyl acetate extract itself extracted with 300 ml. portions of saturated aqueous sodium chloride. The ethyl acetate extract was dried, decolorized with charcoal, filtered.

and the solvent removed from the filtrate in vacuo. A residue weighing about 15 g. was obtained. Preparative TLC over silica indicated three bands (using a 1:2:2 methanol/toluene/ethyl acetate solvent system). Bands containing the desired n-butylether were scraped from the preparative plates, extracted with the same solvent system and rechromatographed using a 1:2 ethyl acetate/toluene solvent system. A final yield of 5.54 g. of 3-O-n-butyl-5,6-benzylidene-L-ascorbic acid was obtained

Mass spectrum: peaks at 320 (molecular ion) 247, 223, 179, 149, 107, 91, 77, 56, 52, 43, 29 and 15.

The following additional compound was obtained by the above procedure:

3-(2-methoxyethyl)-5,6-O-benzylidene-L-ascorbic acid (Compound 32)

Analysis calculated: C, 59.62; H, 5.63;

Found: C, 59.33; H, 5.49.

Mass spectrum: peaks at 149, 91, 77, 59, 44, 30; minor peaks at 322 ($M^+$) 281, 247, 223, 174 and 18.

Example 3

Alternate preparation of

3-O-n-Butyl-L-ascorbic acid (Compound 1)

About 0.5 g. of 3-O-n-butyl-5,6-O-benzylidene-L-ascorbic acid from Example 2 were dissolved in 200 ml. of glacial acetic acid. Five ml. of water were then added and the subsequent reaction mixture stirred at ambient temperature. After about 1.5 hours, TLC indicated that about 50-60% of the starting material still remained; the reaction mixture was therefore allowed to stir at ambient temperature for an additional 48 hours. TLC then indicated that the conversion of the benzylidine derivative to 3-O-n-butyl-L-ascorbic acid was substantially complete. The product was purified by preparative thin layer chromatography over silica using a 1:2:1 methanol/toluene/ethyl acetate eluant. Chemical analysis and other physicochemical measurements indicated that the product of Example 1 had been obtained in pure form.

Example 4

5,6-O-benzylidene-L-ascorbic acid (Compound 33)

Ascorbic acid (89.2 g.) was slurried in 400 ml. of p-dioxane, 200 g. of zinc chloride were added slowly and the resulting mixture was stirred for one hour. Next, 100 ml. (104 g.) of benzaldehyde were added. This reaction mixture was stirred at ambient temperature for about 24 hours and was then extracted

with 500 ml. of ethyl acetate. The ethyl acetate extract was itself extracted with three portions of saturated aqueous sodium chloride. The ethyl acetate solution was dried and the dried solution treated with activated charcoal and then filtered through cellulose. Concentration of the filtrate caused 5,6-O-benzylidene-L-ascorbic acid to crystallize.

Analysis calculated: C, 59.09; H, 4.58;
Found: C, 59.19; H, 4.34.

Yield = 18.3 g.

Other acetals prepared by the above procedure include

5,6-O-(2-phenylethylidene)-L-ascorbic acid (Compound 34)

Analysis calculated: C, 60.4; H, 5.1;
Found: C, 60.3; H, 5.2.

Infrared spectrum: ν at 3258, 1755, 1664 $cm^{-1}$

Mass spectrum: $M^+$ = 278

5,6-O-undecylidene-L-ascorbic acid (Compound 35)

Infrared spectrum; ν at 1665, 1750, 2840, 2920 $cm^{-1}$

Titration: pKa = 6.48

Mass spectrum: $M^+$ = 327

Example 5

5,6-O-(1-methylethylidene)-L-ascorbic acid (Compound 36)

A reaction mixture was prepared from 88 g. of L-ascorbic acid, 400 ml. of dioxane, 200 g. of zinc chloride and 500 ml. of acetone. The reaction mixture was stirred at ambient temperature overnight, and then was washed over a silica 60 column using a toluene-methanol (1:1) solution as the eluant. 600 ml. of wash were collected and the solvent removed therefrom in vacuo. Acetone was added and the solid product filtered. The collected crystals were washed with toluene. A yield of 35.6 g. of 5,6-O-(1-methylethylidene)-L-ascorbic acid was recovered. The compound had the following physical characteristics:

Infrared spectrum: ν at 1670, 1760, 3000, 3250 $cm^{-1}$.

Titration: pKa = 6.10

Mass spectrum: peaks at 216 ($M^+$), 201.

Following the above procedure, these ketals were prepared.

5,6-O-(1-chloromethylethylidene)-L-ascorbic acid (Compound 37)

Analysis calculated: C, 43.1; H, 4.4; O, 38.3; Cl, 14.2;

Found: C, 43.4; H, 4.5; O, 38.2; Cl, 13.9.

Titration: pKa = 6.10

Mass spectrum: peaks at 250 ($M^+$), 201

Infrared spectrum: ν at 1675, 1775, 3000, 3300 $cm^{-1}$

5,6-O-(1-benzyl-2-phenylethylidene)-L-ascorbic acid (Compound 38)

Analysis calculated: C, 68.5; H, 5.4;

Found: C, 68.2; H, 5.6.

Infrared spectrum: ν at 1660, 1740 $cm^{-1}$

Titration: pKa = 6.55.

Mass spectrum: peaks at 369, 354, 277

Example 6

Preparation of 3-O-n-octadecyl-5,6-O-(1-methylethylidene)-L-ascorbic acid (Compound 39)

A reaction mixture was prepared from 20 g. of 5,6-O-(1-methylethylidine)-L-ascorbic acid, 5 g. of sodium methylate, 30.9 g. of n-octadecylbromide and 400 ml. of DMSO. The reaction mixture was stirred at ambient temperature for about five days. Water and ethyl acetate were then added and the ethyl acetate

layer separated. The desired 3-O-n-octadecyl ether contained in that layer was purified by the procedure of Example 1. Chromatography yielded about 1.62 g. of purified 3-O-n-octadecyl-5,6-O-(1- methylethylidene)-L-ascorbic acid.

Analysis calculated: C, 69.2; H, 10.3;

Found: C, 69.2; H, 10.6.

Infrared spectrum: ν at 1705, 1760, 2870, 2930 $cm^{-1}$

Titration: pKa = 11.4

Mass spectrum: peaks at 468, 453

Other ketals preparable by the above procedure include

3-O-(2,5-dimethoxyphenacyl)-5,6-O-(1-methylethylidene)-L-ascorbic acid (Compound 40)

Titration: pKa = 10.59

Infrared spectrum: ν at 1700, 1750, 3340 $cm^{-1}$

Mass spectrum: peaks at 394, 379.

3-O-(2-phthalimidoethyl)-5,6-O-(1-methylethylidene)-L-ascorbic acid (Compound 41)

Titration: pKa = 10.32

Mass spectrum: peaks at 389, 374.

Infrared spectrum: ν at 1710, 1780, 3220 $cm^{-1}$

3-O-(ethoxycarbonylmethyl)-5,6-O-(1-methylethylidene)-L-ascorbic acid (Compound 42)

Infrared spectrum: ν at 1700, 1760, 3000, 3340 $cm^{-1}$

Titration: pKa = 9.80

Mass spectrum: peaks at 302, 287

3-O-(2-ethoxyethyl)-5,6-O-(1-methylethylidene)-L-ascorbic acid (Compound 43)

Titration: pKa = 10.31

Mass spectrum: peaks at 288, 273

Infrared spectrum: ν at 1695, 1765, 2990 $cm^{-1}$

3-O-(2-bromoethoxyethyl)-5,6-O-(1-methylethylidene)L-ascorbic acid (Compound 44)

Analysis calculated: C, 42.5; H, 5.2;

Found: C, 42.7; H, 5.4.

Titration: pKa = 10.4

Mass spectrum: peaks at 368, 353

Infrared spectrum: ν at 1700, 1770, 3010, 3300 $cm^{-1}$

2,3-di-O-n-octadecyl-5,6-O-(1-methylethylidene)-L-ascorbic acid (Compound 45)

No titratable group

Mass spectrum: 721 ($M^+$)

3,4-bis-O-(4-cyanobutyl)-5,6-O-(1-methylethylidene)-L-ascorbic acid (Compound 46)

No titratable group

Infrared spectrum: ν at 1690, 1750, 2260, 3000 $cm^{-1}$

Mass spectrum: peaks at 378, 363

2,3-bis-O-(4-fluorobenzyl)-5,6-O-(1-methylethylidene)-L-ascorbic acid (Compound 47)

Infrared spectrum: ν at 1690, 1765, 2905, 2940, 3005, 3065 $cm^{-1}$

No titratable group.

Mass spectrum: peaks at 432, 214

3-O-(4-nitrobenzyl)-5,6-O-(1-methylethylidene-L-ascorbic acid (Compound 48)

Titration: pKa = 10.10

Mass spectrum: peaks at 351, 336

Infrared spectrum: ν at 1700, 1770, 3360, 3420 $cm^{-1}$

3-O-(3-phenoxypropyl)-5,6-O-(1-methylethylidene)-L-ascorbic acid (Compound 49)

Analysis calculated: C, 61.7; H, 6.3;

Found: C, 59.9; H, 5.7.

Infrared spectrum: ν at 1700, 1780, 3380, 3420 $cm^{-1}$

Titration: pKa = 10.7

Mass spectrum: peaks 350, 335

3-O-n-octadecyl-5,6-O-(1-chloromethylethylidene)-L-ascorbic acid (Compound 50)

Analysis calculated: C, 64.5; H, 9.4; O, 19.1; Cl, 7.1;

Found: C, 64.5; H, 9.5; O, 19.0; Cl, 7.3.

Titration: pKa = 9.0

Mass spectrum: peaks at 502, 453

Infrared spectrum: ν at 1705, 1775, 2860, 2940, 3040 $cm^{-1}$

3-O-n-pentadecyl-5,6-O-(1-methylethylidene)-L-ascorbic acid (Compound 51)

Infrared spectrum: ν at 1710, 1780, 2870, 2940 $cm^{-1}$

Titration: pKa = 10.9

Mass spectrum: peaks at 426, 411.

2,3-di-O-n-pentadecyl-5,6-O-(1-methylethylidene)-L-ascorbic acid (Compound 52)

No titratable groups.

Infrared spectrum: ν at 1690, 1770, 2885, 2940, $cm^{-1}$.

Mass spectrum: peaks at 636, 621.

3-O-(3-fluorobenzyl)-5,6-O-(1-methylethylidene)-L-ascorbic acid (Compound 53)

Analysis calculated: C, 59.3; H, 5.3; F, 5.9;

Found: C, 59.1; H, 5.1; F, 5.6.

Infrared spectrum: ν at 1705, 1760, 3320 $cm^{-1}$

Mass spectrum: peaks at 324, 309

2,3-bis-O-(4-cyanobenzyl)-5,6-O-(1-methylethylidene)-L-ascorbic acid (Compound 54)

Mass spectrum: peaks at 446, 431

No titratable groups.

Infrared spectrum: ν at 1690, 1780, 2250, 2910, 3000 $cm^{-1}$

2,3-bis-O-(2-methylbenzyl)-5,6-O-(1-methylethylidene-L-ascorbic acid (Compound 55)

Infrared spectrum: ν at 1705, 1780, 2950, 3020 $cm^{-1}$

No titratable group.

Mass spectrum: peaks at 424, 409.

3-O-(11-hydroxyundecyl)-5,6-O-(1-methylethylidene)-L-ascorbic acid (Compound 56)

Infrared spectrum: ν at 1710, 1780, 2950, 3540 $cm^{-1}$

Titration: pKa = 10.79

Mass spectrum: $M^+$ 387

3-O-(4-cyanobutyl)-5,6-O-(1-methylethylidene)-L-ascorbic acid (Compound 57)

Titration: pKa = 10.40

Infrared spectrum: ν at 1700, 1765, 3000, 3515 $cm^{-1}$

Mass spectrum: peaks at 297, 282

3-O-methyl-5,6-O-(1-methylethylidene)-L-ascorbic acid (Compound 58)

Infrared spectrum: ν at 1700, 1770 $cm^{-1}$

$^1H$ nmr: δ at 1.3-1.4 (2 singlets, 6H); 3.7-4.5 (multiplet, 7H)

3-O-n-butyl-5,6-O-(1-methylethylidene)-L-ascorbic acid (Compound 59)

Infrared spectrum: ν at 1700, 1770 $cm^{-1}$

$^1H$ nmr: δ at 0.82 (triplet, 3H); 1.3-1.8 (multiplet, 10H); 3.9-4.52 (multiplet, 6H)

3-O-n-hexyl-5,6-O-(1-methylethylidene)-L-ascorbic acid (Compound 60)

Infrared spectrum: ν at 1700, 1770 $cm^{-1}$

$^1H$ nmr: τ at 0.6 (2 singlets, 6H); 1.3-1.6 (multiplet, 12H); 4.65-4.7 (doublet, 1H)

3-O-n-decyl-5,6-O-(1-methylethylidene)-L-ascorbic acid (Compound 61)

Mass spectrum: peaks at 356, 345

Infrared spectrum: ν at 1700, 1770 $cm^{-1}$

$^1H$ nmr: τ at 0.5 (2 singlets, 6H); 1.3-1.7 (multiplet, 20H); 4.65-4.7 (doublet, 1H)

3-O-(2-methoxyethyl)-5,6-O-(1-methylethylidene-L-ascorbic acid (Compound 62)

Infrared spectrum: ν at 1770, 1770 $cm^{-1}$

$^1H$ nmr: δ at 1.3-1.4 (2 singlets, 6H); 5.38 (singlet, 3H); 3.6-4.72 (multiplet, 8H).

Example 7

Preparation of 2-O-Benzyl-3-O-n-hexadecyl-L-ascorbic acid (Compound 63)

A solution of .933 g. of 3-O-n-hexadecyl-L-ascorbic acid in 7.5 ml. of anhydrous DMF was added slowly at ambient temperature under a nitrogen atmosphere to a suspension of 2.45 millimoles of NaH in 10 ml. of anhydrous DMF in a 50 ml., 3-neck round-bottom flask equipped with magnetic stirrer, drying tube and dropping funnel. The reaction mixture was stirred for 25 minutes (until $H_2$ evolution stopped) at which time the sodium salt (on the 2-hydroxy) of 3-O-n-hexadecyl-L-ascorbic acid had formed. A solution of 0.295 g. of benzyl chloride in 2 ml. of anhydrous DMF was added. The reaction mixture was stirred at ambient temperature for about 50 minutes. The reaction temperature was then raised to 90°C., at which temperature stirring was continued for an additional 50 minutes. The reaction mixture was cooled and saturated aqueous sodium chloride (brine) added. The resulting aqueous mixture was extracted with ethyl acetate. The ethyl acetate extract was washed with brine and dried. The dried extract was decolorized with charcoal, filtered and the volatile constituents removed in vacuo. The resulting yellow syrup was chromatographed over silica gel 60 using 1:9 ethyl acetate-toluene as the eluant. Fractions shown by TLC to contain the desired product were combined and the solvent removed therefrom. 694

mg. (62% yield) of a yellow waxy solid comprising purified 2-O-benzyl-3-O-n-hexadecyl-L-ascorbic acid were obtained.

Analysis calculated: C, 70.99; H, 9.45;
Found: C, 71.05; H, 9.63.

Proton magnetic resonance spectrum: $\delta$ at 7.35 (singlet-5H), 5.1 (singlet-2H).

Mass spectrum: peaks at 490 ($M^+$), 459, 398, 338, 295, 177, 116, 91.

Infrared spectrum: $\nu$ at 1761, 1672 $cm^{-1}$

As previously stated, the compounds of this invention inhibit the action of angiogenesis factor in promoting the development of blood vessels (as part of the growth process) by tumors, by which mechanism the tumor is able to form an adequate blood supply system. One method of demonstrating such angiogenesis factor inhibitory action in vivo is by the following test procedure.

Lysosomal-mitochondrial pellets containing angiogenesis factor are prepared from 3683 Morris hepatoma. The pellet is diluted with 7-8 ml. of 15% ficoll. With this dilution, 8-10 serpentine vessels will be produced per standard of dye as injection of the lysosomal-mitochondrial pellet. The dilution may be adjusted upward or downward to bring the number of serpentine vessels induced within the 8-10 range so as to provide comparable concentrations of angiogenesis factor per lysosomal-mitochondrial preparation.

Next, 15SPF/ND4 female mice weighing 20-22 g. are shaved on the left side and then divided into three groups of five each. One group is injected subcutaneously and laterally with 0.20 cc. of the lysosomal-mitochondrial preparation diluted with 15% ficoll. This group of mice is then dosed individually by the intraperitoneal route with 0.5 cc. of a solution or suspension in a standard vehicle containing the compound under test, usually at an initial dose level of 300 mg./kg. If this dose level is toxic, two-fold dilutions are made until all mice survive a single dose. The second group of mice is injected subcutaneously and laterally with 0.2 cc. of the lysosomal-mitochondrial suspension diluted with ficoll and dosed intraperitoneally with 0.5 cc. of the vehicle alone. The mice are sacrificed after 24 hours. Each mouse is placed on its side on a dissecting board with the shaved side up. Starting at the flank, the skin is cut straight to the back of the animal. A similar cut is made behind the front leg. Then the skin is cut along the back making a flap of about one inch by two inches. The skin is carefully separated from the connective tissue using forceps and a scalpel. The skin flap is then laid back exposing the lysosomal-mitochondrial implant which is attached to the skin. The skin flap is gently flattened out and with the use of a binocular dissecting scope, serpentine vessels are observed around the lysosomal-mitochondrial implant and their number

counted. All observations of the number of serpentine vessels are made at the same power of the microscope (1X). The average number of serpentine vessels for each group is calculated. The percent inhibition is then calculated according to the following equation.

$$\%\ \text{inhibition} = \left(1 - \frac{\text{sv (Control group)}}{\text{sv (drug group)}}\right) \times 100$$

where sv = average number of serpentine vessels.

Tables I, II and III which follow give the results of these tests.

Table I covers compounds according to formula I above in which $R^7$ and $R^8$ are both H, table II covers compounds in which $R^7$ and $R^8$ form a 1-methylethylidene group, and Table III compounds in which $R^7$ plus $R^8$ equal a benzylidine group or other group.

One of the compounds of this invention, 3-O-n-octadecyl-5,6-O-(1-methylethylidene)-L-ascorbic acid was tested at a series of dose levels for its ability to inhibit tumor angiogenesis and the results of these tests are set forth in Table IV.

Table 1

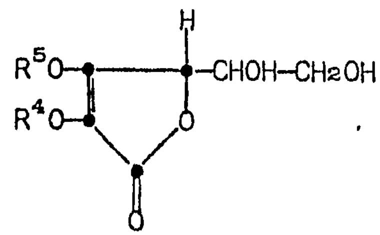

(IX)

| Compound | $R^5$ | $R^4$ | average %inhibition | Range dose mg./kg. |
|---|---|---|---|---|
| 2 | 2,6-dichlorobenzyl | H | 56 | 150-300 |
| 5 | n-dodecyl | H | 59 | 25-300 |
| 6 | 3-bromobenzyl | H | 74 | 300 |
| 7 | 3-fluorobenzyl | H | 52 | 25 |
| 8 | 10-carboxy-n-decyl | H | 41 | 25 |
| 9 | n-pentadecyl | H | 50 | 300 |
| 10 | n-pentadecyl | n-pentadecyl | 38 | 25-300 |
| 11 | 2-bromoethoxyethyl | H | 36 | 300 |
| 12 | 3-phenoxypropyl | H | 68 | 300 |
| 13 | 2-phthalimidoethyl | H | 55 | 300 |
| 14 | n-hexadecyl | H | 31 | 25 |
| 15 | n-hexadecyl | n-hexadecyl | 13 | 25-150 |
| 17 | n-octadecyl | H | 82 | 25-300 |
| 18 | n-octadecyl | n-octadecyl | 52 | 25 |
| 21 | 3-chlorobenzyl | H | 41 | 25 |
| 22 | 4-chlorobenzyl | H | 36 | 25-300 |
| 23 | 3-trifluoromethylbenzyl | H | 53 | 25-300 |
| 24 | 3-methylbenzyl | H | 54 | 25 |
| 25 | 2,5-dimethylbenzyl | H | 47 | 25-300 |
| 30 | 2-chlorobenzyl | H | 55 | 25 |

Table 2

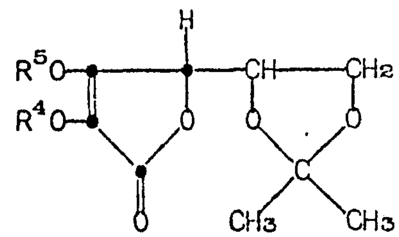

(X)

| Compound | $R^5$ | $R^4$ | average % inhibition | Range dose mg./kg. |
|---|---|---|---|---|
| 36 | H | H | 48 | 10 |
| 39 | n-octadecyl | H | 38-82 | 25-300 |
| 41 | 2-phthalimidoethyl | H | 30 | 120 |
| 42 | ethoxycarbonylmethyl | H | 12 | 10 |
| 44 | 2-bromoethoxyethyl | H | 71 | 240 |
| 45 | n-octadecyl | n-octadecyl | 18-85 | 25 |
| 46 | 4-cyanobutyl | 4-cyanobutyl | 47-82 | 25-150 |
| 47 | 4-fluorobenzyl | 4-fluorobenzyl | 45 | 37.5 |
| 48 | 4-nitrobenzyl | H | 42-85 | 150 |
| 49 | 3-phenoxypropyl | H | 36 | 150 |
| 51 | n-pentadecyl | H | 15-85 | 25-150 |
| 52 | n-pentadecyl | n-pentadecyl | 15-85 | 25-150 |
| 53 | 3-fluorobenzyl | H | 27-82 | 25 |
| 54 | 4-cyanobenzyl | 4-cyanobenzyl | 36-91 | 25 |
| 56 | 11-hydroxyundecyl | H | 67 | 150 |
| 57 | 4-cyanobutyl | H | 37-72 | 37.5-150 |
| 58 | methyl | H | 15 | 10 |
| 59 | n-butyl | H | 60 | 10 |
| 60 | n-hexyl | H | 41 | 10 |
| 61 | n-decyl | H | 48 | 10 |
| 62 | 2-methoxyethyl | H | 28-61 | 10-240 |

Table 3

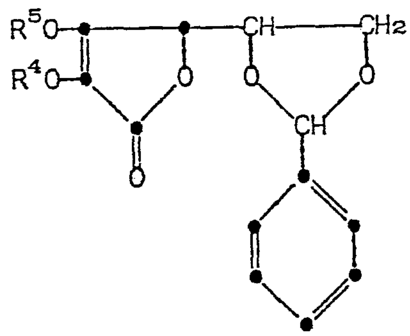

(XI)

| $R^5$ | $R^4$ | % inhibition |
|---|---|---|
| n-butyl | H | 60 |
| 2-methoxyethyl | H | 31 |

*150 mg./kg. dose intraperitoneally

Table 4

Evaluation of 3-O-n-octadecyl-5,6-O-(1-methylethylidene)-L-ascrobic acid

| Intraperitoneal dose mg./kg. | % inhibition | |
|---|---|---|
| 240 | 71, 78 | = 74.5 |
| 120 | 66, 78, 75, 71 | = 72.5 |
| 60 | 72, 50 | = 62.5 |
| 30 | 58, 38 | = 48 |
| 15 | 45, 17 | = 32 |

In addition, the compounds of this invention have found utility as angiogenesis inhibitors in metastasis formation. Such activity has been found in an artificial metastasis model using Madison lung (M 109) carcinoma which metastasizes preferentially to lungs and is not overly sensitive to chemotherapeutic agents. The test is carried out as follows.

MADISON LUNG METASTASIS ASSAY

The Madison (M109) lung carcinoma is carried as a transplantable line in syngeneic BALB/C mice. This tumor line was obtained from the tumor bank at Mason Research Institute, Worcester, Mass. For tumor metastasis studies, a subcutaneously-grown tumor is aseptically excised, minced into small pieces with a scissors, and gently trypsinized at room temperature to obtain a single cell suspension. The cells are suspended in RPMI-1640 medium (M. A. Bioproducts, Walkersville, MD). Viable M109 cells are determined by trypan blue exclusion, and the cell concentration is determined with a hemocytometer. The cells are adjusted to 1 x $10^5$ viable cells/ml. of medium. M109 cells are injected i.v. into normal, male BALB/C mice. Inoculum volume per mouse is 0.2 ml. (2 x $10^4$ cells). Drugs are administered i.p. to randomized groups of 10 mice 2 days prior to tumor cell inoculation. Controls receive mock injections of 0.5 ml. buffer. Daily mortality is monitored, and median survival times are determined for each group. The results of this test on 3-O-n-octadecyl-L-ascorbic

acid are recorded in Table 5. Cytoxan was employed as a positive control. In the Table, column 1 gives drug treatment and columns 2 and 3 measure number of lesions ± standard error per lung on days 30 and 42.

Table 5

| Drug Treatment | Mean Number of Lesions ± S.E./Lung | |
|---|---|---|
| | Day 30 | Day 42 |
| Emulphor control | 15.8 ± 4.6 | 20.6 ± 1.8 |
| Cytoxan (30 mg./kg.)* | 8.4 ± 1.5 | --- |
| 3-O-n-octadecyl-L-ascorbic acid (35 mg./kg.) | 1.8 ± 1.2 | 18.6 ± 1.3 |
| 3-O-n-octadecyl-L-ascorbic acid (35 mg./kg.) + cytoxan (30 mg./kg.) | 1.6 ± 0.6 | toxic |

*Cytoxan given every 4th day I.P. starting on day 12.

The growth rate and number of lung metastasis in the above experiment were less than usual. A new transplant line was employed in a second determination with lung lesions developing earlier. Table 6 records the results of this experiment; here ascorbic acid was employed as the control.

Table 6

| Drug Treatment** | Mean No. of Lesions ± S.E./Lung 16 day |
|---|---|
| Emulphor control | 69.8 ± 10.4 |
| ascorbic acid (100 mg./kg.) | 33.8 ± 9.6 |
| 3-O-n-octadecyl-L-ascorbic acid (30 mg./kg.) | 10.7 ± 3.4 |
| 3-O-n-octadecyl-L-ascorbic acid (100 mg./kg.) | 13.0 ± 5.1 |

**All drugs given daily from day 0.

The compounds useful in this invention are relatively non-toxic, having $LD_{50}$'s above 400 or 1000 mg./kg. in mice.

Another laboratory test involving angiogenesis or neovascularization is based on the time period required for a differentiated tumor to become undifferentiated (vascularized). An inflammatory response enhances tumor growth and reduces the lag phase. In this test, rats are injected with a test drug (30 minutes prior to dosing) with ICFA (incomplete Freund's adjuvant) plus India ink intradermally in a shaved area on a rat's back, thus providing a marked injection site. After 3 days of twice daily administration of the test drug followed 30 minutes later by administration of ICFA, the tumor is transplanted on the periphery of the

marked injection site. The animals are weighed and the tumor size determined (length + width/2) at weekly intervals for 4 weeks. Morris hepatoma (5123D) was used as the undifferentiated tumor.

Following the above experimental procedure, 10-100 mg. of 3-O-n-octadecyl-L-ascorbic acid administered once or twice a day orally either inhibited the growth of the undifferentiated tumor or delayed its induction from 4-7 days. 0.5 cc. of ICFA were also given once or twice daily subcutaneously to each test rat.

A third laboratory test has been employed to demonstrate the activity of compounds according to I above as inhibitors of angiogenesis. This test method is a collagen arthritis assay carried out as follows.

Type II collagen is isolated from bovine articular cartilage by the method of Strawich and Nimni [Biochemistry, 10, 3905, (1971)]. The collagen is dissolved in 0.1 M acetic acid and stored at -20°. Type II collagen solution is diluted to 2 mg./ml. concentration and emulsified thoroughly with an equal volume of incomplete Freund's adjuvant (ICFA). The emulsion containing approximately 0.5 mg. of collagen is injected intradermally to groups of 6 inbred Lewis male rats (Charles River Breeders; 170-200 g.) at various sites in the dorsal area. The hindpaw volumes of each rat is measured and recorded three times a week throughout the test period to assess the inflammatory reaction. The animals receive compounds

under test as suspensions in carboxymethylcellulose vehicle, by oral gavage, 5 days per week (Mon.-Fri.). At the end of the test (day 28 or 30), the blood of these animals is drawn by cardiac puncture and the serum anti-type II collagen antibody levels are estimated by passive hemagglutination technique, using glutaraldehyde treated sheep red cells, to which type II collagen is conjugated [Avrameas et al., Immunochemistry, 6, 67, (1969); Andriopoulos et al., Arth. Rheum., 19, 613, (1976)]. The cellular response or delayed-type hypersensitivity response to type II collagen is measured by the radiometric ear index assay (Kostiala, Immunology, 33, 561, 1977). In certain experiments, the bone damage occurring because of immunization with type II collagen and the effects of drugs are determined from the radiographs of the hindpaws of two or three representative animals from each group. Injections of ICFAs alone were employed to some rats as a negative control.

In one experiment carried out according to the above protocol, 3-O-n-octadecyl-5,6-O-(1-methylethylidine)-L-ascorbic acid and 3-O-n-octadecyl-Lascorbic acid were the test drugs and were administered orally at a 50 mg./kg. dose. The former compound inhibited the swelling of the paw caused by injection of Type II collagen by about 50% and with the latter, paw volumes were not substantially different from those of the ICFA-treated rats (negative controls). In other runs, with 3-O-n-octadecyl-L-ascorbic acid, at the 50 mg./kg. dose level, paw volumes were 90-100% lower than those of rats immunized with type II collagen but not treated with drugs (positive controls). With 3-O-n-octadecyl-5,6-O-(1-methylethylidene)-L-ascorbic acid at the same dose level, the paw volumes were indistinguishable from negative control levels.

At lower dosages with 3-O-n-octadecyl-L-ascorbic acid, a 12.5 mg./kg. dose decreased paw volumes by about 25%, but at a 17.5 mg./kg. dose, the paw volumes were indistinguishable from controls.

2,3-O-bis(n-octadecyl)-L-ascorbic acid at 12.5 and 25 mg./kg. dose levels also gave decreased paw volumes (33-67%). With 3-O-(m-trifluoromethylbenzyl)-L-ascorbic acid at a 25 mg./kg. dose, paw volumes were substantially the same as ICFA controls.

The following additional drugs gave substantial decreases in paw swelling caused by collagen Type II injections at a drug dose of 15 mg./kg. per os: 3-O-n-heptadecyl-L-ascorbic acid, 2,3-O-bis(4-cyano-benzyl)-5,6-(1-methylethylidene)-L-ascorbic acid, 3-O-(4-cyanobutyl)-5,6-(1-methylethylidene)-L-ascorbic acid, and 5,6-O-(1-n-decylethylidene)-L-ascorbic acid.

In utilizing the compounds of this invention as angiogenesis inhibiting agents, either the parenteral or oral route of administration may be employed. For oral dosage, which is preferred, a suitable quantity of a drug according to formula I is mixed

with one or more conventional, pharmaceutically acceptable excipients such as starch and the mixture placed in telescoping gelatin capsules, each capsule containing a divided or undivided dose. Alternatively, a mixture of the drug plus starch plus a lubricant plus other pharmaceutically acceptable excipients as required is compressed into tablets each containing from 100 to 500 mg. of active ingredient. The tablets may be scored if lower or divided dosages are to be used. For parenteral administration, the drug is administered in solution or suspension. Each unit dose of the drug for either route of administration will contain an amount of a drug according to I above effective to inhibit angiogenesis. Daily dose levels in mammals would be in the range 10-100 mg./kg. of mammalian body weight.

## Claims

1. A compound of the formula (I)

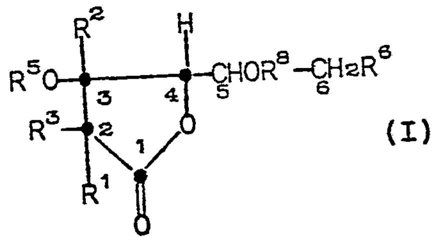

(I)

wherein:

$R^1$ and $R^2$ are both hydrogen or combine to form a second bond between $C_2$ and $C_3$,

$R^3$ is OH, $NH_2$ or $OR^4$,

$R^4$ and $R^5$ are independently selected from

$(C_8-C_{22})$alkyl,

$-CH_2(C_2-C_{12})$alkenyl,

$-CH_2(C_2-C_{12})$alkynyl,

$-(C_1-C_{21})$alkyl-X-$(C_1-C_{21})$alkyl,

wherein X is O, CO, S, NH, $N(C_1-C_5)$alkyl, SO, or $SO_2$, or

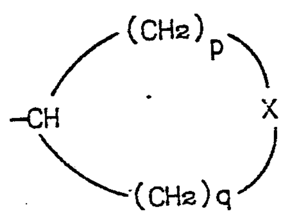

wherein X is as defined above and the sum of p and q is 1-6, said $R^4$ and $R^5$ groups being unsubstituted or optionally substituted with one or two groups independently selected from Cl, Br, F, I, $(C_1-C_5)$-alkoxycarbonyl, phenoxy, OH, $CF_3$, $(C_1-C_5)$alkoxy, nitro, -CN, $-SO_3H$, $-PO_3H_2$, di-$(C_1-C_5)$-alkylamino, or phthalimido,

$R^6$ is H, F or $OR^7$;

$R^7$ and $R^8$, taken singly, are independently selected from H, $(C_1-C_{12})$alkyl, and benzyl, or, taken together, are

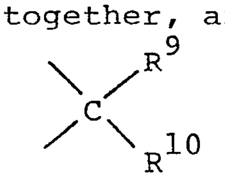

wherein $R^9$ and $R^{10}$ are independently selected from

H,

a $(C_1-C_{10})$alkyl group that is optionally substituted with halo, phenyl, or substituted phenyl, said optional phenyl substituents being one or two groups independently selected from halo, hydroxy, $(C_1-C_5)$alkoxy, nitro, $CF_3$, and $(C_1-C_5)$alkyl, or

optionally substituted phenyl, wherein said optional phenyl substituents are as defined above,

provided that only one of $R^9$ and $R^{10}$ can be H, or a pharmaceutically acceptable salt thereof.

2. The compound of claim 1 wherein $R^1$ and $R^2$ combine to form a second bond between $C_2$ and $C_3$.

3. The compound of claim 2 which is an ascorbic acid or isoascorbic acid derivative.

4. The compound of claim 3 which is an L-ascorbic acid derivative.

5. The compound of any one of claims 1-4 wherein $R^4$ or $R^5$ is $(C_8-C_{22})$alkyl.

6. The compound of any one of claims 1-5 wherein $R^6$ is $OR^7$ and both $R^7$ and $R^8$ are hydrogen.

7. The compound of any one of claims 1-5 wherein $R^6$ is $OR^7$ and $R^7$ and $R^8$ combine to form

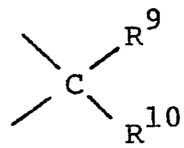

8. The compound of claim 7 wherein $R^9$ is hydrogen.

9. A process for preparing a compound of the formula (I)

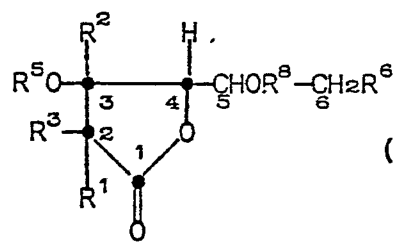

(I)

wherein:

$R^1$ and $R^2$ are both hydrogen or combine to form a second bond between $C_2$ and $C_3$,

$R^3$ is OH, $NH_2$ or $OR^4$,

$R^4$ and $R^5$ are independently selected from
$(C_8-C_{22})$alkyl,
$-CH_2(C_2-C_{12})$alkenyl,
$-CH_2(C_2-C_{12})$alkynyl,
$-(C_1-C_{21})$alkyl-X-$(C_1-C_{21})$alkyl,

wherein X is O, CO, S, NH, $N(C_1-C_5)$alkyl, SO or $SO_2$, or

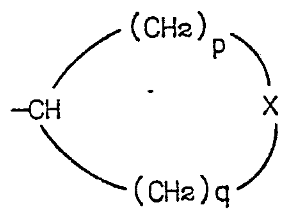

wherein X is as defined above and the sum of p and q is 1-6, said $R^4$ and $R^5$ groups being unsubstituted or optionally substituted with one or two groups independently selected from Cl, Br, F, I, $(C_1-C_5)$-alkoxycarbonyl, phenoxy, OH, $CF_3$, $(C_1-C_5)$alkoxy, nitro, -CN, $-SO_3H$, $-PO_3H_2$, di-$(C_1-C_5)$-alkylamino, or phthalimido,

$R^6$ is H, F or $OR^7$;

$R^7$ and $R^8$, taken singly, are independently selected from H, $(C_1-C_{12})$alkyl, and benzyl, or, taken together, are

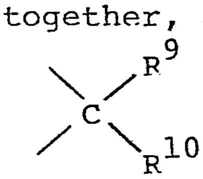

wherein $R^9$ and $R^{10}$ are independently selected from

H,

a $(C_1-C_{10})$alkyl group that is optionally substituted with halo, phenyl, or substituted phenyl, said optional phenyl substituents being one or two groups independently selected from halo, hydroxy, $(C_1-C_5)$alkoxy, nitro, $CF_3$, and $(C_1-C_5)$alkyl, or

optionally substituted phenyl, wherein said optional phenyl substituents are as defined above,

provided that only one of $R^9$ and $R^{10}$ can be H;

which comprises the step of:

(a) reacting a compound of the formula (II)

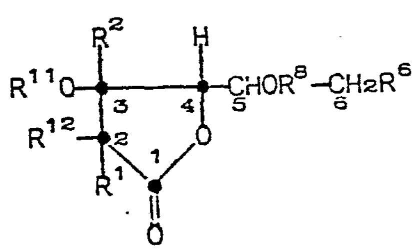

(II)

wherein $R^1$, $R^2$, $R^6$ and $R^8$ are as defined above, $R^{11}$ is H or $R^5$ as defined above, and

$R^{12}$ is OH, $OR^4$ as defined above, or $NH_2$, provided that $R^{12}$ is OH if $R^{11}$ is other than H, with an alkylating reagent of the formula $R^4Z$ or $R^5Z$, where Z is a leaving group, in the presence of a base, or

(b) when $R^{11}$ is other than H, $R^6$ is $OR^7$, and $R^7$ and $R^8$ combine to form

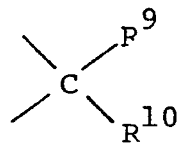

,

subjecting the compound of formula II to acid hydrolysis to provide a product of formula I wherein $R^7$ and $R^8$ are hydrogen.

10. The method of claim 9 wherein $R^4$ or $R^5$ is $(C_8-C_{22})$alkyl.

11. A compound of the formula (I)

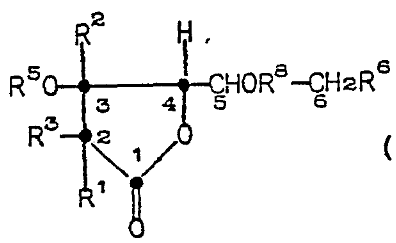

(I)

wherein:

$R^1$ and $R^2$ are both hydrogen or combine to form a second bond between $C_2$ and $C_3$,

$R^3$ is OH, $NH_2$ or $OR^4$,

$R^4$ and $R^5$ are independently selected from

$(C_1-C_{22})$alkyl,

$-CH_2(C_2-C_{12})$alkenyl,

$-(CHR^{13})_m-Y-R^{14}$ wherein m is 0 to 12, Y is O, S, or a direct bond, $R^{13}$ is H or $(C_1-C_5)$alkyl, and $R^{14}$ is $(C_3-C_8)$cycloalkyl, $(C_3-C_8)$cycloalkenyl, $(C_7-C_{12})$bicycloalkyl, $(C_7-C_{12})$-bicycloalkenyl, or aryl,

$-CH_2(C_2-C_{12})$alkynyl,

$-(C_1-C_{21})$alkyl-X-$(C_1-C_{21})$alkyl, wherein X is O, CO, S, NH, $N(C_1-C_5)$alkyl, SO or $SO_2$, or

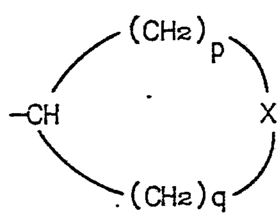

wherein X is as defined above and the sum of p and q is 1-6, said $R^4$ and $R^5$ groups being unsubstituted or optionally substituted with one or two groups independently selected from Cl, Br, F, I, $(C_1-C_5)$-

alkoxycarbonyl, phenoxy, OH, $CF_3$, $(C_1-C_5)$alkoxy, nitro, -CN, $-SO_3H$, $-PO_3H_2$, di-$(C_1-C_5)$-alkylamino, or phthalimido,

$R^6$ is H, F or $OR^7$;

$R^7$ and $R^8$, taken singly, are independently selected from H, $(C_1-C_{12})$alkyl, and benzyl, or, taken together, are

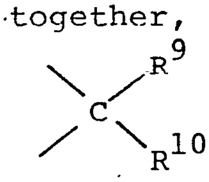

wherein $R^9$ and $R^{10}$ are independently selected from

H,

a $(C_1-C_{10})$alkyl group that is optionally substituted with halo, phenyl, or substituted phenyl, said optional phenyl substituents being one or two groups independently selected from halo, hydroxy, $(C_1-C_5)$alkoxy, nitro, $CF_3$, and $(C_1-C_5)$, or

optionally substituted phenyl, wherein said optional phenyl substituents are as defined above,

provided that only one of $R^9$ and $R^{10}$ can be H, and the pharmaceutically acceptable salts thereof, for use as a pharmaceutical.

12. A compound of any one of claims 1-8 for use as a pharmaceutical.

13. A compound of any one of claims 1-8 and 11 for use as an angiogenesis inhibiting agent.

14. A pharmaceutical formulation including, as an active ingredient, a compound of the formula (I)

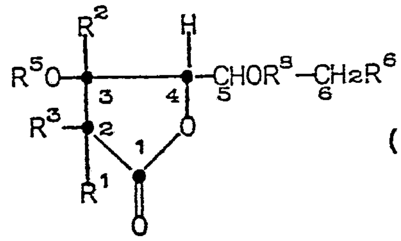

(I)

wherein:

$R^1$ and $R^2$ are both hydrogen or combine to form a second bond between $C_2$ and $C_3$,

$R^3$ is OH, $NH_2$ or $OR^4$,

$R^4$ and $R^5$ are independently selected from

$(C_1-C_{22})$alkyl,

$-CH_2(C_2-C_{12})$alkenyl,

$-(CHR^{13})_m-Y-R^{14}$ wherein m is 0 to 12, Y is O, S, or a direct bond, $R^{13}$ is H or $(C_1-C_5)$alkyl, and $R^{14}$ is $(C_3-C_8)$cycloalkyl, $(C_3-C_8)$cycloalkenyl, $(C_7-C_{12})$bicycloalkyl, $(C_7-C_{12})$-bicycloalkenyl, or aryl,

$-CH_2(C_2-C_{12})$alkynyl,

$-(C_1-C_{21})$alkyl-X-$(C_1-C_{21})$alkyl, wherein X is O, CO, S, NH, $N(C_1-C_5)$alkyl, SO or $SO_2$, or

wherein X is as defined above and the sum of p and q is 1-6, said $R^4$ and $R^5$ groups being unsubstituted or optionally substituted with one or two groups independently selected from Cl, Br, F, I, $(C_1-C_5)$-alkoxycarbonyl, phenoxy, OH, $CF_3$, $(C_1-C_5)$alkoxy, nitro, -CN, $-SO_3H$, $-PO_3H_2$, di-$(C_1-C_5)$-alkylamino, or phthalimido,

$R^6$ is H, F or $OR^7$;

$R^7$ and $R^8$, taken singly, are independently selected from H, $(C_1-C_{12})$alkyl, and benzyl, or, taken together, are

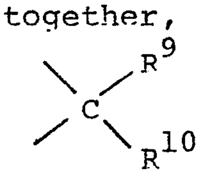

wherein $R^9$ and $R^{10}$ are independently selected from

H,

a $(C_1-C_{10})$alkyl group that is optionally substituted with halo, phenyl, or substituted phenyl, said optional phenyl substituents being one or two groups independently selected from halo, hydroxy, $(C_1-C_5)$alkoxy, nitro, $CF_3$, and $(C_1-C_5)$, or

optionally substituted phenyl, wherein said optional phenyl substituents are as defined above,

provided that only one of $R^9$ and $R^{10}$ can be H, or a pharmaceutically acceptable salt thereof associated with one or more pharmaceutically acceptable excipients or carriers therefore.

15. A pharmaceutical formulation including, as an active ingredient, a compound of any one of

BAD ORIGINAL

claims 1-8, associated with one or more pharmaceutically acceptable excipients or carriers therefor.

Claims

1. A process for preparing a compound of the formula (I)

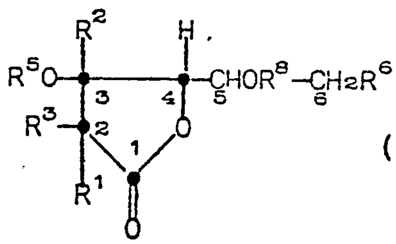

(I)

wherein:

$R^1$ and $R^2$ are both hydrogen or combine to form a second bond between $C_2$ and $C_3$,

$R^3$ is OH, $NH_2$ or $OR^4$,

$R^4$ and $R^5$ are independently selected from

$(C_8-C_{22})$alkyl,

$-CH_2(C_2-C_{12})$alkenyl,

$-CH_2(C_2-C_{12})$alkynyl,

$-(C_1-C_{21})$alkyl-X-$(C_1-C_{21})$alkyl,

wherein X is O, CO, S, NH, $N(C_1-C_5)$alkyl, SO or $SO_2$, or

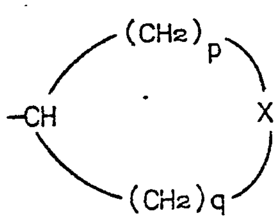

wherein X is as defined above and the sum of p and q is 1-6, said $R^4$ and $R^5$ groups being unsubstituted or optionally substituted with one or two groups independently selected from Cl, Br, F, I, $(C_1-C_5)$-alkoxycarbonyl, phenoxy, OH, $CF_3$, $(C_1-C_5)$alkoxy, nitro, -CN, $-SO_3H$, $-PO_3H_2$, di-$(C_1-C_5)$-alkylamino, or phthalimido,

$R^6$ is H, F or $OR^7$;

$R^7$ and $R^8$, taken singly, are independently selected from H, $(C_1-C_{12})$alkyl, and benzyl, or, taken together, are

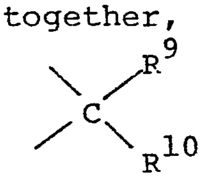

wherein $R^9$ and $R^{10}$ are independently selected from

H,

a $(C_1-C_{10})$alkyl group that is optionally substituted with halo, phenyl, or substituted phenyl, said optional phenyl substituents being one or two groups independently selected from halo, hydroxy, $(C_1-C_5)$alkoxy, nitro, $CF_3$, and $(C_1-C_5)$alkyl, or

optionally substituted phenyl, wherein said optional phenyl substituents are as defined above,

provided that only one of $R^9$ and $R^{10}$ can be H;

which comprises the step of:

(a) reacting a compound of the formula (II)

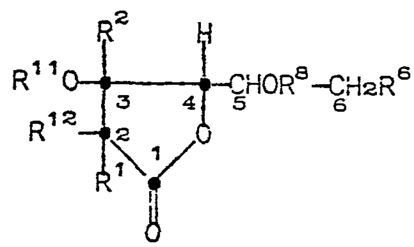

(II)

wherein $R^1$, $R^2$, $R^6$ and $R^8$ are as defined above, $R^{11}$ is H or $R^5$ as defined above, and

$R^{12}$ is OH, $OR^4$, or $NH_2$, provided that $R^{12}$ is OH if $R^{11}$ is other than H, with an alkylating reagent of the formula $R^4Z$ or $R^5Z$, where Z is a leaving group, and $R^4$ and $R^5$ are as defined above, in the presence of a base, or

(b) when $R^{11}$ is other than H, $R^6$ is $OR^7$, and $R^7$ and $R^8$ combine to form

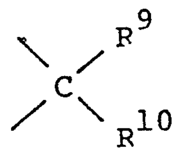

,

subjecting the compound of formula (II) to acid hydrolysis to produce a product of formula I wherein $R^7$ and $R^8$ are hydrogen.

2. The process of claim 1 wherein $R^1$ and $R^2$ combine to form a second bond between $C_2$ and $C_3$.

3. The process of claim 2 wherein the reactant of formula II is ascorbic acid or isoascorbic acid or a derivative of ascorbic acid or isoascorbic acid.

4. The process of claim 3 wherein the reactant of formula II is L-ascorbic acid or a derivative thereof.

5. The process of any one of claims 1-4 wherein $R^4$ or $R^5$ is $(C_8-C_{22})$alkyl.

6. The process of any one of claims 1-5 wherein $R^6$ is $OR^7$ and both $R^7$ and $R^8$ are hydrogen.

7. The process of any one of claims 1-5 wherein $R^6$ is $OR^7$ and $R^7$ and $R^8$ combine to form

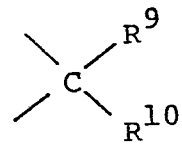

8. The process of claim 7 wherein $R^9$ is hydrogen.

9. A compound of formula (I) whenever prepared by a process according to any one of claims 1-8.

0086554

European Patent Office

# EUROPEAN SEARCH REPORT

Application number

EP 83 30 0174

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | CHEMICAL ABSTRACTS, vol. 40, no. 21, 10th November 1946, cols. 6591,6592, Columbus Ohio (USA); C.S.VESTLING et al.: "The antiscorbutic properties of 3-methylascorbic acid". & J. BIOL. CHEM. 164, 631-7(1946). *Abstract* | 11-15 | C 07 D 307/62<br>C 07 D 407/04<br>C 07 D 405/12<br>C 07 D 405/14<br>A 61 K 31/375 |
| A | CHEMICAL ABSTRACTS, vol. 29, no. 1, 10th January 1935, cols. 737,738, Columbus Ohio (USA); W.N.HAWORTH et al.: "Methyl ethers of ascorbic acid". *Abstract* | 1-10 | |
| P | CARBOHYDRATE RESEARCH, vol. 102, 1982, pages 302-307, Elsevier Scientific Publishing Company, Amsterdam (NL); H.A.PARISH, Jr. et al.: "The reaction of 1,1-dibromopinacolone with L-ascorbic acid". *The whole article, particularly page 303, formula 3* | 1 | TECHNICAL FIELDS SEARCHED (Int. Cl. 3)<br>C 07 D 307/00<br>C 07 D 405/00<br>C 07 D 407/00<br>A 61 K 31/00 |

The present search report has been drawn up for all claims

| Place of search | Date of completion of the search | Examiner |
|---|---|---|
| THE HAGUE | 13-04-1983 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82